# EUROPEAN PATENT APPLICATION

(11) **EP 1 669 084 A1**
(43) Date of publication of application: **14.06.2006**
(21) Application number: 04773639.2
(22) Date of filing: 30.09.2004
(51) Int. Cl.: A61K 38/16, A61K 38/36, A61K 38/01, A61P 19/08, A61P 19/10, A23L 1/305, A23K 1/16

(54) **AGENT FOR PROMOTING OSTEOGENESIS AND/OR INHIBITING BONE RESORPTION**

(30) Priority: 30.09.2003 JP 2003340803; 30.09.2003 JP 2003340804; 27.07.2004 JP 2004218786
(71) Applicant: SNOW BRAND MILK PRODUCTS CO., LTD., Sapporo, Hokkaido 065-0043 (JP)
(72) Inventor: MORITA, Yoshikazu, Kawagoe-shi, Saitama 350-1165 (JP); SERIZAWA, Atsushi, Kawagoe-shi, Saitama 350-1165 (JP); MATSUYAMA, Hiroaki, Kawagoe-shi, Saitama 350-1165 (JP); MOTOURI, Mutsumi, Kawagoe-shi, Saitama 350-1165 (JP); KAWAKAMI, Hiroshi, Kawagoe-shi, Saitama 350-1165 (JP)
(74) Representative: Wakerley, Helen Rachael
(86) International application number: PCT/JP2004/014761
(87) International publication number: WO 2005/030244

(57) **Abstract**

Provided is a novel agent for promoting osteogenesis and/or inhibiting bone resorption, which: can be directly taken from daily meals by mouth for long periods without any trouble in taste; directly impart a promoting effect on osteogenesis and an inhibiting effect on bone resorption to the bone; and can be expected to have therapeutic effects for preventing or ameliorating/treating various bone diseases. The above agent includes, as an active ingredient, any one or more of β₂-microglobulin, histone, complement component 3, monocyte chemotactic protein1, lysozyme, ribonuclease, prothrombin, cytochrome P-450, plasminogen, transferrin, thrombin, plasmin, complement component 4, and β defensin, and enzymatic digestion products thereof. Those substances have a remarkable inhibiting effect on bone resorption by osteoclasts and/or a promoting effect on osteogenesis via promotion of the proliferation and differentiation of osteoblasts.

## Description

### [Technical Field]

The present invention relates to an agent for promoting osteogenesis and/or inhibiting bone resorption, which includes, as an active agent, any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, β defensin, and enzymatic digestion products thereof. In addition, the present invention relates to an agent for promoting osteogenesis and/or inhibiting bone resorption and foods, drinks, drugs, or feeds for promoting osteogenesis and/or inhibiting bone resorption, combined with any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, β defensin, and enzymatic digestion products thereof.

### [Background Art]

In recent years, with an increase in the elderly population, various bone diseases such as osteoporosis, bone fracture, and lumbago tend to increase. In the bone tissue, osteogenesis and bone resorption constantly occur and are being balanced in youth. However, the balance shifts to the bone resorption owing to various causes involved in aging (uncoupling). Also, continuance of this state for a long period of time makes the bone tissue fragile, resulting in occurrence of various bone diseases such as osteoporosis, bone fracture, and lumbago will be caused. Inhibition of the uncoupling may prevent various bone diseases such as osteoporosis, bone fracture, and lumbago.

Approaches, which have been conventionally carried out to inhibit the uncoupling and to prevent or treat various bone diseases, include: (1) the dietary intake of calcium; (2) light exercises; (3) insolation; and (4) medication,and the like. For the dietary intake of calcium, calcium salts such as calcium carbonate and calcium phosphate and natural calcium agents such as eggshells and fish bone powders have been used. However, those materials are not always appropriate for oral ingestion.

As for the light exercise, taking a jog or walk is believed to be appropriate. However, even the light exercise poses a threat to a person whose body is being weakened. Besides, the bed-ridden elderly can hardly exercise. The insolation is believed to be good for the supplying of activated vitamin D3, but it is not complete in itself. For the medication, for example, 1α-hydroxyvitamin D3, calcitonin preparations, and the like have been used and also known to be effective in the therapy of osteoporosis. However, those materials are definitely medicines themselves and not available as food materials.

Osteoclasts are cells that develop from hematopoietic stem cells and reside on the surface of cancellous bone to dissolve the bone. In addition, osteoblasts reside on the surface of bone tissue and play a central role in osteogenesis in which bone matrix proteins such as collagen are actively synthesized. On the bone matrix proteins, calcium phosphate or hydroxyapatite crystals can be deposited (calcification) to make hard bone tissue.

The formation and growth of bone (modeling) and the metabolism (remodeling) thereof may occur such that the osteoclasts dissolve the bone matrix (bone resorption) and then the osteoblasts synthesize the bone matrix (osteogenesis).

The inventors of the present invention have searched an osteogenesis promoting factor and a bone resorption inhibiting factor in whey protein to obtain substances that have an osteogenesis promoting effect and a bone resorption inhibiting effect and can be used as food materials. As a result, The inventors of the present invention have found out a bone strengthening effect in a protein and peptide mixture prepared by removing salts of whey origin from a water-soluble fraction of whey protein by treatment with reverse osmotic membrane, electrodialysis, or the like (JP-A-04-183371). Furthermore, they have found an osteoblast proliferation promoting effect and a bone strengthening effect in a fraction obtained by subjecting an aqueous solution of the protein and peptide mixture to an ethanol treatment, a heat treatment, a salting treatment, and an ultrafiltration membrane treatment (e.g., JP-A-05-176715 and JP-A-05-320066). In addition, they have found an osteoblast proliferation promoting effect, a bone strengthening effect, and a bone resorption preventing effect in a basic protein present in minute amounts in milk (JP-A-08-151331).

The inventors of the present invention have conducted a further search and made an attempt to isolate and purify the active principle body having a promoting effect on osteogenesis and an inhibiting effect on bone resorption to identify such a substance. As a result, The inventors of the present invention have found out that β₂-microglobulin, histone, complement component 3, monocyte chemotactic protein 1, lysozyme, ribonuclease, prothrombin, cytochrome P-450, plasminogen, transferrin, thrombin, plasmin, and enzymatic digestion products thereof, which are known substances, can markedly inhibit the bone resorption by osteoclasts.

In addition, the inventors have found that the osteogenesis can be facilitated by promoting the proliferation of osteoblasts as the bone resorption by osteoclasts is markedly inhibited by prothrombin, cytochrome P-450, plasminogen, transferrin, thrombin, plasmin, and enzymatic digestion products thereof.

Furthermore, the present invention has been completed by finding out that osteogenesis can be promoted by facilitating the differentiation of osteoblasts by complement component 4, β defensin, and enzymatic digestion products thereof.

β₂-microglobulin is a protein of about 11. 6 kDa in molecular weight, a constituent of the Major Histocompatibility Complex (MHC). β₂-microglobulin is known to be widely distributed in tissues all over the body as well as blood, and the presence thereof in a human being and bovine milk is also reported. As for the effect of β₂-microglobulin on the bone, an increase in blood level of β₂-microglobulin as a marker of bone metabolic turnover for high-turnover osteoporosis has been reported (J. M. Quesada and six other persons, "Serum β₂-microglobulin as a Marker for High Bone Remodeling of Elderly Woman ", Mechanism of Aging and Development, (U.S.) 1998, No. 102, p. 293-298). In addition, there is another report in which β₂-microglobulin was able to promote osteogenesis as it promoted the proliferation of cultured osteoblasts MC3T3 (E. Balint and two other persons, "Role of interleukin-6 in β₂-microglobulin induced bone mineral dissolution", Kidney International, (U.S.), 2000, No. 57, p1599-1607) . On the other hand, as for the action of bone resorption, there is a report in which β₂-microglobulin promotes the elution of calcium from bone (J. Petersen and another person, "In vivo Effect of β₂-microglobulin on Bone Resorption", American Journal of Kidney Diseases, (U.S.), 1994, No. 23, p726-730). However, the promoting effect on the elusion of calcium is different from the inhibiting effect on bone resorption. There is no literature that describes the action of β₂-microglobulin to inhibit the bone resorption as has been found by The inventors of the present invention.

Histones are basic proteins found in the cellular nuclei of higher animals. They are essential proteins to effectively house DNA (deoxyribonucleic acid) which controls genetic information and is said to be of about 2 m in straight distance in a cellular nucleus for a higher animal. In other words, DNA is functionally folded while being wound around histones. In general, histones include five different molecular species, H1, H2A, H2B, H3, and H4. Eight molecules in total, which contain two of each molecular species H2A, H2B, H3, and H4, form a single aggregate. DNA is wound around the aggregate to form a unit referred to as a chromatin. H1 has a function of allowing chromatins to adhere together. In this way, histones and DNA are always in pairs and present at a weight ratio of approximately 1 : 1 in nucleus. As for DNA, until now, various reports for nutritional effects of nucleic acids and components thereof have been conducted. Of those, there is an idea that the nucleic acid may be the sixth essential nutrient. As for histones coupled with DNA, in contrast, there is no report for the nutritional effects. Foods of animal origin are basically cellular aggregates. Thus, so long as the foods are taken, histones are the proteins inevitably taken without depending on animal species. In the case of humans, however, they have extremely many opportunities to take a cellular food products prepared using refined foodmaterials having no cellular nucleus, such as polished rice, wheat flour, and sugar. If only the bone resorption preventing effects of histones are expected, food materials such as liver and milt serving as cellular aggregates may be prepared in diets in excess. In this case, however, there are great many restrictions because of large problems in taste and food processing.

Complement components are enzyme-like substances present in serum and activated by reaction with an antigen-antibody complex to exert physiological activities while evoking complicated reactions. Currently, nine components C1 to C9 are known. Complement component 3 corresponds to C3 and is referred to as complement third component, the most abundant one in complement components. It consists of a glycoprotein in which an α-chain having a molecular weight of 105, 000 and a β-chain having a molecular weight of 75,000 are cross-linked by S-S bonds at two different positions. Furthermore, C3 convertase can divide the C3 of 180, 000 in molecular weight into C3a of 9,000 in molecular weight caused by partial decomposition of the α-chain and the remaining C3b of 170,000 in molecular weight having the S-S bonds at two different portions. C3a is known as one of the representative anaphylatoxins that provoke inflammatory reactions such as one in which mast cells are stimulated to promote the discharge of histamine. C3b can be further cleaved by C3 convertase and a part thereof is then covalently bonded to an antigen-antibody complex. Phagocytes such as macrophages in blood have C3b receptors on their cell surfaces, so that accordingly the reaction can urge the phagocytes to phagocytize foreign substances. In this manner, complement component 3 (C3) is intimately-involved in immunoreactions. In addition, nutritional factor compositions for embryos and fetuses and for tissue cultures thereof and compositions for infertility treatment, which contain complement component 3 as an active ingredient, have been disclosed (JP-A-10-033164). For osteoclasts having functions of decomposing the bone tissue as the self tissue, the present invention has revealed that complement component 3 serves as an inhibitor for the activation of such a function.

Monocyte Chemotactic Protein-1 or Monocyte Chemoattractant Protein-1 (MCP-1) is a monocyte chemotactic factor produced by monocytes, capillary endothelium, glioma cell lines, or the like. It is one of the representative C-C chemokine family members with RANTES (regulated on activation, normal T cell expressed and secreted) and is of 76 amino acids with a molecular weight of 8, 000 to 18, 000, mainly representing chemotaxis to monocytes but not acting on neutrophils and lymphocytes.

Lysozyme is an enzyme that hydrolyzes β-1-4-linkages between N-acetylmuramic acid and N-acetylglucosamine present in mucopeptide and the like on the bacterial cell wall and is widely distributed in animal and plant kingdoms. It can be divided into four groups on the basis of substrate specificity and molecular weight.

Ribonuclease is a generic term for enzymes that acts on ribonucleic acid (RNA), one of the nucleic acids, to cause mononuleotides or oligonucleotides.

Prothrombin is a protein of about 7.2 kDa in molecular weight and also referred to as blood coagulation factor II. It is one of the vitamin-K dependent blood coagulation factors and can be decomposed into thrombin by factor X in the presence of vitamin K. The resulting thrombin breaks fibrinogen into fibrin and the fibrin is then cross-linked, thereby causing the coagulation of blood. Prothrombin is known to be widely distributed in tissues all over the body as well as blood, and the presence thereof in a human being and bovine milk is also reported.

As described above, thrombin is a product obtained by decomposing prothrombin with an enzyme such as factor X.

Cytochrome P-450 is a generic term of the family of protohem proteins in reduced form, which binds carbon monoxide and shows a Soret absorption band in the vicinity of 450 nm. Functionally, it contributes to: synthesis and decomposition reactions of various kinds of steroid hormone, bile acids, and prostanoids; reactions for ω-oxidation of fatty acids and activation of vitamin D; and oxidative detoxication reactions for innumerable foreign pharmacological agents and environmental pollutants or the like taken in the body.

Plasminogen is a precursor of plasmin present in the plasma of animals, and a plasminogen activator cleaves Arg-Val bond in a plasminogen molecule to form active plasmin. Plasminogen is a single-stranded glycoprotein of about 91,000 in molecular weight, and protease for plasmin or the like cleaves a peptide on the N-terminal side to form modified plasminogen of about 82,000 in molecular weight.

In this way, plasmin is one obtained by decomposing plasminogen using an enzyme such as the plasminogen activator.

Transferrin is a protein of about 75, 000 in molecular weight, which binds to transport iron in blood, also referred to iron-binding globulin. Immature erythrocytes have receptors for transferrin-bound iron. For allowing serum iron to be utilized in the synthesis of hemoglobin, serum iron should be combined with transferrin. Thus, with regard to transferrin, there is disclosed that a membrane-bound transferrin-like protein promote the formation of chondrocytes. However, it is for promoting the formation of chondrocytes, so that the mechanism thereof is completely different from one for promoting osteogenesis (JP-A-2002-20311 and WO 01/013951).

Complement component 4 (C4) is an enzyme-like protein present in serum and activated by reaction with an antigen-antibody complex to exert physiological activities while evoking complicated reactions. Complement component C4 itself has no specificity to antigen but is activated by the antigen-antibody complex to cause the acceleration of phagocytic action and destroy cells or bacteria bound to the specific antibody. Complement component 4 is a protein consisting of three different polypeptide chains, α-, β-, and γ-chains, and having a molecular weight of 210 kDa. The promoting effect of complement component 4 on the differentiation of osteoblasts has not been known.

Defensin is known as a highly-basic bactericidal peptide having three S-S linkages in the molecule. In other words, it is a basic protein consisting of 29 to 34 amino acids having 4-10 arginine residues and 6 cysteine residues. From a positional difference in cysteine residues being located, defensin can be mainly grouped into α- and β-types. As well as the antibacterial activities for gram-positive bacteria and gram-negative bacteria, defensin retains the antibacterial activities for molds and viruses. β-defensin is expressed on a respiratory epithelial cell or a mucosa epithelial cell, and six types thereof have been known for a human being. In addition to the antibacterial action, it is known that defensin is involved in a wide variety of biophylaxis, such as cytotoxicity for normal cells and cancer cells, histamin release action from mast cells, and monocyte chemotaxis. A therapeutic agent for retrovirus infectious disease where filtrates from specific culture media of yeast and lactic acid bacteria are administered for the secretion of defensin or the like (JP-A-2004-115497), a cosmetic composition made of roots of *Artemisia princeps* that stimulate the development of defensin in skin (JP-A-2004-067660), and so on, have been disclosed. However, there is no knowledge about the promotion of osteoblast differentiation.

Of the substances having those osteogenesis promoting effects and bone resorption inhibiting effects, as described above, there is a report describing that, for example, β₂-microglobulin may promote the elution of calcium from bone. However, as also described above, the promoting effect on the.elution of calcium is different from the inhibiting effect on the resorption of bone, so that there is no literature describing an effect that β₂-microglobulin inhibits bone resorption, like the one found out by the inventors of the present invention. In addition to β₂-microglobulin, furthermore, there is no literature that describes the function of any one of histone, complement component 3, monocyte chemotactic protein 1, lysozyme, ribonuclease, prothrombin, cytochrome P-450, plasminogen, transferrin, thrombin, plasmin, complement component 4, β defensin, and enzymatic digestion products thereof to promote osteogenesis and inhibit bone resorption.

### [Disclosure of the Invention]

### [Problems to be solved by the Invention]

An object of the present invention is to provide a novel agent for promoting osteogenesis and inhibiting bone resorption which: can be directly taken from daily meals by mouth for long periods without any trouble in taste on the basis of the nature of various bone diseases such as osteoporosis, bone fracture, and lumbago; directly impart a promoting effect on osteogenesis and an inhibiting effect on bone resorption to the bone; and can be expected to have therapeutic effects for preventing or various bone diseases such as osteoporosis, bone fracture, and lumbago, and to provide foods, drinks, drugs, or feeds for promoting osteogenesis and inhibiting bone resorption.

### [Means for solving the Problems]

In consideration of those problems, the inventors of the present invention have dedicated themselves to study substances showing bone-strengthening effectswidely included infood materials and made an attempt to isolate and purify a component having a promoting effect on osteogenesis and an inhibiting effect on bone resorption and then identified such a substance, with respect to a protein having a promoting effect on osteogenesis and an inhibiting effect on bone resorption. Then, they have found out that: β₂-microglobulin, histone, complement component 3, monocyte chemotactic protein 1, lysozyme, ribonuclease, prothrombin, cytochrome P-450, plasminogen, transferrin, thrombin, plasmin, and enzymatic digestion products thereof, which are known substances, can markedly inhibit the bone resorption by osteoclasts; the osteogenesis can be facilitated by promoting the proliferation of osteoblasts as the bone resorption by osteoclasts is markedly inhibited by prothrombin, cytochrome P-450, plasminogen, transferrin, thrombin, plasmin, and enzymatic digestion products thereof; and furthermore the osteogenesis can be promoted by facilitating the differentiation of osteoblasts by complement component 4, β defensin, and enzymatic digestion products thereof.

Furthermore, the present invention has been completed by finding out that those substances can be used as agents for promoting osteogenesis and/or inhibiting bone resorption.

That is, the present invention relates to an agent for promoting osteogenesis and/or inhibiting bone resorption, including: any one or more of β₂-microglobulin, histone, complement component 3, monocyte chemotactic proteinl,lysozyme, ribonuclease, prothrombin, cytochrome P-450, plasminogen, transferrin, thrombin, plasmin, complement component 4, and β defensin, and enzymatic digestion products thereof as an active ingredient.

The present invention relates also to a food, drink, drug, or feed for promoting osteogenesis and/or inhibiting bone resorption, including: any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin, and enzymatic digestion products thereof.

### [Effects of the Invention]

An agent for promoting osteogenesis and/or inhibiting bone resorption of the present invention includes, as an active ingredient, any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin, and enzymatic digestion products thereof, and is useful for preventing or ameliorating/treating various bone diseases such as osteoporosis when orally administered. In addition, when those active ingredients are blended in foods, drinks, drugs, and feeds, a promoting effect on osteoenesis or an inhibiting effect on bone resorption can be exerted for preventing or alleviating various bone diseases such as osteoporosis.

### [Best Mode for carrying out the Invention]

The present invention relates to an agent for inhibiting bone resorption, which is blended with any one or more of β₂-microglobulin, histone, complement component 3, monocyte chemotactic protein 1, lysozyme, ribonuclease, prothrombin, cytochrome P-450, plasminogen, transferrin, thrombin, and plasmin, and enzymatic digestion products thereof to inhibit bone resorption of osteoclasts.

Furthermore, the present invention relates to an agent for promoting osteogenesis and inhibiting bone resorption, which contains any one or more of prothrombin, cytochrome P-450, plasminogen, transferrin, thrombin, plasmin and enzymatic digestion products thereof, thereby significantly inhibiting the bone resorption of osteoclasts and promoting the proliferation of osteoblasts.

Furthermore, the present invention relates to an agent for promoting osteogenesis, which contains any one or more of complement component 4, β defensin, and enzymatic digestion products thereof to promote the differentiation of osteoblasts.

The balance of bone metabolism can be expected to be shifted to the dominance of osteogenesis by taking advantage of the osteogenesis promoting effects and/or bone resorption inhibiting effects, which are found in β₂-microglobulin, histone, complement component 3, monocyte chemotactic protein 1, lysozyme, ribonuclease, prothrombin, cytochrome P-450, plasminogen, transferrin, thrombin, plasmin, complement component 4, β defensin, and enzymatic digestion products thereof. Therefore, the present invention is able to provide an agent for promoting osteogenesis and/or inhibiting bone resorption, foods, drinks, drugs, or feeds for promoting osteogenesis and/or inhibiting bone resorption, each of which has a promoting effects on osteogenesis and an inhibiting effect on bone resorption.

In the present invention, any of β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin each of which is used as an active ingredient for promoting osteogenesis and/or inhibiting bone resorption in the present invention is present in minute amounts in milk of bovines, water buffalos, humans, pigs, sheep, goats, horses, and the like, and can be separated and extracted from the milk. For a milk constituent, a protein fraction separated from raw milk, skimmilk, or whey or a cellular fraction in milk separated from raw milk can be used. β₂-microglobulin, complement component 3, monocyte chemotactic protein 1, prothrombin, cytochrome P-450, plasminogen, transferrin, thrombin, plasmin, complement component 4, and β defensin can be also prepared from the blood of healthy domestic animals. For raw materials for the extraction of histone, grain embryos, milts, and the like can be used as well as the milk constituent. For the grain embryos, wheat embryos, rice embryos, and the like can be used. The milts used may be those collected from fishes and shellfishes, including salmon, trout, codfish, herring, squid, and scallop. In particular, milts of fishes and shellfishes which can be caught in large amounts such as salmon, trout, and codfish are preferably used in terms of effective utilization of resources.

Any of β₂-microglobulin, histone, complement component 3, monocyte chemotactic proteinl,lysozyme, ribonuclease, prothrombin, cytochrome P-450, plasminogen, transferrin, thrombin, plasmin, complement component 4, and β defensin is commercially available, so that a commercial product thereof maybe also used. Alternatively, a genetically-engineered recombinant product may be used.

Furthermore, one obtained by the decomposition of β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin with proteases such as trypsin, pancreatin, chymotrypsin, pepsin, papain, kallikrein, cathepsin, thermolysin, and V8 protease may be used. For instance, the cleavage product of prothrombin is a peptide mixture obtained by partially cleaving the above prothrombin by a protease. Thrombin obtained by cleaving prothrombin by an enzyme such as factor X may be used. Alternatively, plasmin obtained by cleaving plasminogen by an enzyme such as a plasminogen activator may be used. Thrombin and plasmin are commercially available as well as prothrombin and plasminogen, so that the commercial products thereof can be used.

As for preparation from milk, for instance, fresh milk may be brought into contact with an ion-exchange resin to adsorb fractions that contain β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin, respectively, and the fractions are then eluted by gradually rising the level of sodium chloride, followed by obtaining each of them by gel filtration chromatography.

For preparation from blood, citric acid plasma is reacted with barium citrate and then respective precipitates of β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin adsorbed to insoluble barium are collected. Furthermore, their purities can be enhanced by ion exchange chromatography.

The agent for promoting osteogenesis and/or inhibiting bone resorption of the present invention includes, as an active ingredient, any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, β defensin, and enzymatic digestion products thereof. In addition, any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, β defensin, and enzymatic digestion products thereof may be combined with foods and drinks such as cow milk, milk beverages, coffee drinks, juices, jellies, cookies, breads, noodles, and sausages, or may be provided as drugs in tablets, powders, or other forms. Furthermore, calcium preparations each having good absorbency, such as calcium chloride, calcium carbonate, calcium lactate, and calcium originated from egg shells and milk, may be used together to shift the balance of bone metabolism to the dominance of osteogenesis, thereby further raising a strengthening effect on bone.

The dose of the agent for promoting osteogenesis and/or inhibiting bone resorption of the present invention varies among an active ingredient, age, therapeutic effect, pathologic condition, and the like. In the case of a human adult, 1 ng to 1 g per day may be divided and taken orally. In this way, by taking the agent for promoting osteogenesis and/or inhibiting bone resorption of the present invention, various bone diseases such as osteoporosis can be prevented. Furthermore, any of β₂ microglobulin, histone, complement component 3, monocyte chemotactic protein 1, lysozyme, ribonuclease, prothrombin, cytochrome P-450, plasminogen, transferrin, thrombin, plasmin, complement component 4, and β defensin is the original component originated from the plasma or milk, and it did not show any acute toxicity in rats. Furthermore, feeds may contain those active ingredients to promote the osteogenesis of domestic animals, poultry, and the like and also to inhibit the bone resorption thereof.

Hereinafter, the present invention will be described in more detail by way of Examples and Test Examples. However, these examples are only provided for illustrating the embodiments of the invention and thus the present invention is not limited thereto.

### [Test Example 1]

### (Inhibitory effect on bone resorption)

Inhibitory effects on bone resorption were investigated for β₂-microglobulin, histone, complement component 3, monocyte chemotactic protein 1, lysozyme, ribonuclease, prothrombin, cytochrome P-450, plasminogen, transferrin, thrombin, and plasmin, respectively. The respective active ingredients employed in the test were commercially available. The respective active ingredients employed in the test are listed in Table 1 with respect their origins and final concentrations.

The long bone of a postnatal 10 to 20-day ICRmouse was extracted and the soft tissue was then removed therefrom, followed by morcellating the bone mechanically in a solution of α-MEM (manufactured by Flow Laboratories, Co., Ltd.) containing 5% bovine fetal serum to obtain the whole marrow cells including osteoclasts. The whole marrow cells including the osteoclasts were dispersed on the surface of an ivory piece so as to be of about 2 x 10⁶ cells in amounts and spotted with the α-MEM solution containing 5% bovine fetal serum. After two hours, each of β₂-microglobulin, histone, complement component 3, monocyte chemotactic protein 1, lysozyme, ribonuclease, prothrombin, cytochrome P-450, plasminogen, transferrin, thrombin, and plasmin was added with the α-MEM solution containing 5% bovine fetal serum so that its final concentration would be as described in Table 1, and then the whole was incubated for 5 days at 37°C, followed by investigating the inhibitory effects of the existing osteoclasts on bone resorption.

Inhibitoryeffects onbone resorption were evaluated such that, after the incubation, the cells on the ivory piece were peeled off and stained with hematoxylin, followed by counting the number of bone resorption pits by image analysis with PIASLA-555 (Takeshi Seno et al., "Manual of selected cultured cell lines for bioscience and biotechnology",pp.199-200,1993). In other words, a low number of pits means that the bone resorption was inhibited as the activities of osteoclasts decreased. In the pit assay, furthermore, the substance showing the bone resorption inhibiting effect also showed an inhibiting effect on bone resorption in an animal experiment (Toba et al. , Bone, vol. 27, p.403-408, 2000), so that the pit assay can be generally an experimental system suitable for investigating the bone resorption inhibiting effect. One without the addition of an active ingredient was used as a control. The respective bone resorption activities (%) were represented in Table 1 such that the bone resorption activity of the active-ingredient free one was defined as 100%.

The results are shown in Table 1.

**Table 1**

| Active ingredient | Concentration | | Relative activity (%) | | |
|---|---|---|---|---|---|
| Monocyte chemotactic protein 1 (manufactured by Biotech Corp.) | 0.1 | (pg/ml) | 75.4 | ± | 33.8 |
| | 1 | | 45.9 | ± | 22.1 |
| | 10 | | 47.2 | ± | 11.3 |
| Histone H1/2A (Manufactured by Sigma, Co., Ltd) | 10 | (µg/ml) | 79.1 | ± | 26.7 |
| | 100 | | 67.1 | ± | 26.6 |
| Lysozyme (Manufactured by Sigma, Co., Ltd) | 25 | (unit/ml) | 70.6 | ± | 23.7 |
| | 250 | | 42.4 | ± | 21.8 |
| | 2,500 | | 31.5 | ± | 14.4 |
| Ribonuclease (Bovine sperm) | 1 | (µg/ml) | 80.2 | ± | 18.0 |
| | 10 | | 45.6 | ± | 12.2 |
| (Manufactured by Sigma, Co., Ltd) | 100 | | 29.9 | ± | 15.5 |
| Complement third component (Human being) | 0.25 | (µg/ml) | 93.1 | ± | 10.6 |
| | 2.5 | | 81.0 | ± | 25.0 |
| (Manufactured by Sigma, Co., Ltd) | 25 | | 61.7 | ± | 17.4 |
| β₂-microglobulin (Human being) (Manufactured by Sigma, Co., Ltd) | 0.1 | (µg/ml) | 80.9 | ± | 13.9 |
| | 1 | | 37.2 | ± | 16.4 |
| | 10 | | 9.7 | ± | 3.9 |
| Plasminogen (Manufactured by Sigma, Co., Ltd) | 1 | (µg/ml) | 44.1 | ± | 37.9 |
| | 10 | | 13.8 | ± | 19.9 |
| | 100 | | 2.0 | ± | 2.8 |
| Transferrin (Manufactured by Funakoshi Co., Ltd) | 1 | (µg/ml) | 70.7 | ± | 36.6 |
| | 10 | | 51.9 | ± | 19.4 |
| Cytochrome P-450 (Rabbit) (Manufactured by Sigma, Co., Ltd) | 1 | (µg/ml) | 94.7 | ± | 10.2 |
| | 10 | | 83.0 | ± | 27.6 |
| | 100 | | 75.3 | ± | 16.3 |
| Prothrombin (Manufactured by Sigma, Co., Ltd) | 0.1 | (µg/ml) | 62.0 | ± | 19.7 |
| | 1 | | 35.1 | ± | 16.0 |
| | 10 | | 25.1 | ± | 20.1 |
| Plasmin (Manufactured by Sigma, Co., Ltd) | 1 | (µg/ml) | 54.1 | ± | 27.1 |
| | 10 | | 23.3 | ± | 19.1 |
| | 100 | | 5.0 | ± | 2.5 |
| Thrombin (Manufactured by Sigma, Co., Ltd) | 1 | (µg/ml) | 55.0 | ± | 16.5 |
| | 10 | | 39.1 | ± | 13.0 |
| | 100 | | 26.3 | ± | 15.1 |

Any of those added with β₂-microglobulin, histone, complement component3,monocyte chemotactic proteinl,lysozyme,ribonuclease, prothrombin, cytochrome P-450, plasminogen, transferrin, plasmin, and thrombin each serving as an active ingredient of the present invention showed the inhibitory effect on bone resorption, compared with that of the active-ingredient free one. Consequently, it was found that they had significant inhibitory effects on bone resorption.

### [Test Example 2]

### (Proliferation effects on osteoblasts)

Proliferation effects on osteoblasts were investigated for prothrombin, cytochrome P-450, plasminogen, transferrin, thrombin, and plasmin, respectively. The respective active ingredient employed in the test were commercially available. The respective active ingredients employed in the test were listed in Table 2 with respect their origins and final concentrations.

Mouse osteoblstic MC3T3-E1 cells were inoculated in a 96-well plate at a cell number of 2 x 10⁴/ml with an α-MEM medium (manufactured by Flow Laboratories, Co., Ltd.) containing 10% bovine fetal serum and then incubated for 24 hours at 37°C in the presence of 5%CO₂ so as to be provided as test culture cells. Subsequently, the medium was replaced with an α-MEM medium free of bovine fetal serum and then added with any of prothrombin, cytochrome P-450, plasminogen, transferrin, plasmin, and thrombin to its final concentration described in Table 2, followed by incubation for 18 hours at 37°C. The cell proliferation activity thereof was investigated with a cell proliferation assay kit (manufactured by Amersham Biosciences, Co., Ltd.) using bromodeoxyuridine (BrdU). One without the addition of an active ingredient was used as a control. The respective cell proliferation activities (%) were represented in Table 2 such that the cell proliferation activity of the active-ingredient free one was defined as 100%. The results are shown in Table 2.

**Table 2**

| Active ingredient | Concentration | | Relative Relative activity (%) | | |
|---|---|---|---|---|---|
| Plasminogen (Manufactured by Sigma, Co., Ltd) | 0.5 | (µg/ml) | 163 | ± | 21 |
| | 5 | | 235 | ± | 27 |
| Transferrin (Manufactured by Funakoshi | 0.5 | (µg/ml) | 146 | ± | 17 |
| Co., Ltd) | 5 | | 235 | ± | 17 |
| Cytochrome P-450 (Manufactured by Sigma, Co., Ltd) | 0.5 | (µg/ml) | 172 | ± | 15 |
| | 5 | | 199 | ± | 22 |
| | 50 | | 220 | ± | 27 |
| Prothrombin (Manufactured by Sigma, Co., Ltd) | 0.1 | (µg/ml) | 201 | ± | 11 |
| | 1 | | 353 | ± | 2 |
| | 10 | | 379 | ± | 3 |
| Plasmin (Manufactured by Sigma, Co., Ltd) | 0.5 | (µg/ml) | 141 | ± | 10 |
| | 5 | | 206 | ± | 23 |
| Thrombin (Manufactured by Sigma, Co., Ltd) | 0.1 | (µg/ml) | 134 | ± | 9 |
| | 1 | | 165 | ± | 11 |
| | 10 | | 176 | ± | 20 |

Any of those added with prothrombin, cytochrome P-450, plasminogen, transferrin, plasmin, and thrombin showed a significant increase in proliferation activity of mouse osteoblastic MC3T3-E1 cells with a concentration dependency compared with the active-ingredient free one. Consequently, it was found that they had proliferation effects on osteoblasts.

### [Test Example 3]

### (Promotion effect on osteoblast differentiation)

The promotion effects of complement component 4 and β defensin on the differentiation of osteoblasts were investigated. In other words, human-origin pre-osteoblastic MG-63 cells were inoculated in a 96-well plate at a cell number of 2 x 10⁴/ml with a DMEM culture medium (manufactured by Flow Laboratories, Co., Ltd.) containing 10% bovine fetal serum for 4 days at 37°C in the presence of 5% CO₂ so as to be provided as test culture cells. Subsequently, the medium was replaced with a medium containing 1% bovine fetal serum and then added with complement component 4 (Complement component C4, C8195, manufactured by Sigma, Co., Ltd.) to final concentrations of 0.1, 1, and 10 µg/ml and also added with β-defensin (β-defensin 1 and 2, manufactured by Peptide Institute, Inc.) to final concentrations of 0.1, 1, and 10 µg/ml, followed by incubation for 5 days at 37°C. The culture supernatant was collected and the level of collagen type 1 therein was determined with Procollagen Type I C-peptide EIA Kit (Takara MK101, manufactured by Takara Shuzo Co., Ltd.) to investigate its activity of promoting the differentiation of osteoblasts. One free of complement component 4 and β defensin was used as a control. The resulting amount of collagen produced was expressed in a ratio (%) of the measuring amount of type I collagen in each sample to the measuring amount thereof in the control. The results are shown in Table 3.

**Table 3**

| | Final concentration (µg/ml) | The amount of collagen produced (%) |
|---|---|---|
| Control (no addition) | - | 100±6 |
| Complement component 4 | 0.1 | 138±7 |
| | 1 | 169±15 |
| | 10 | 161±9 |
| β defensin 1 | 0.1 | 127±11 |
| | 1 | 183±9 |
| | 10 | 197±18 |
| β defensin 2 | 0.1 | 134±9 |
| | 1 | 147±5 |
| | 10 | 177±14 |

Any of the groups added with complement component 4 and β defensin showed increased levels of type I collagen, compared with those of the control groups (free of complement component 4 and β defensin), thereby revealing that they have the promoting effects on the differentiation of osteoblasts.

As described above, from the test results of "Test Example 1", it is found that the bone resorption of osteoclasts can be significantly inhibited by β₂-microglobulin, histone, complement component 3, monocyte chemotactic protein 1, lysozyme, ribonuclease, prothrombin, cytochrome P-450, plasminogen, transferrin, thrombin, plasmin, and enzymatic digestion products thereof. In addition, from the test results of "Test Example 2", it is found that any of prothrombin, cytochrome P-450, plasminogen, transferrin, thrombin, plasmin, and enzymatic digestion products thereof can promote osteogenesis by enhancing the proliferation of osteoblasts while inhibiting the bone resorption of osteoclasts. Furthermore, from the test results of "Test Example 3", any of complement component 4, β defensin, and enzymatic digestion products thereof promotes osteogenesis by promoting the differentiation of osteoblasts.

### [Example 1]

### (β₂-microglobulin-containing agent for inhibiting bone resorption)

According to a known method (Hoshi F., D. Nagai, S. Higuchi, T. Noso, A. Takahasi, S. Kawamura, Veterinary Immunology and Immunopathology, 53, 29-38, 1996. Purification of bovine beta 2-microglobulin from colostrums and its complete amino acid sequence), β₂-microglobulin was prepared from bovine colostrum.

In other words, skimmilk was obtained from 10 liters of bovine colostrum by means of centrifugation and then adjusted to pH 4 with 1N hydrochloric acid, followed by precipitation of aggregated casein by means of centrifugation. Twenty grams of the casein was dissolved in one liter of a 5-mM sodium biphosphate buffer (PBS, pH 8.3) and then adsorbed on a diethylaminoethyl (DEAE) cellulose (DE-52, manufactured by Whatman, Co., Ltd.) column being equilibrated with the same buffer. The resultant was washed with an equilibration buffer and the resulting fraction was then analyzed on SDS-polyacryl amide gel electrophoresis (SDS-PAGE). Subsequently, the fraction containing a 12-kDa protein was concentrated through ultrafiltration, followed by overnight dialysis at 4°C against 5 mM PBS (pH 8.3). The concentrate was passed through gel filtration chromatography of Sephadex G-75 column equilibrated with 5 mM PBS (pH 8.3). The fraction containing a 12-kDa protein was concentrated through ultrafiltration and then dialyzed against a 10-mM ammonium acetate solution. The resultant was freeze-dried, thereby resulting in 12 mg of β₂-microglobulin, an active ingredient of the present invention. Twelve miligrams of the resulting β₂-microglobulin was mixed with 120 mg of lactose and formulated into granules, thereby resulting in an agent for inhibiting bone resorption of the present invention.

Furthermore, any of histone, complement component 3, monocyte chemotactic protein 1, lysozyme, ribonuclease, prothrombin, cytochrome P-450, plasminogen, transferrin, thrombin, plasmin, complement component 4, and β defensin can be also prepared from bovine colostrum by the same way as that of β₂-microglobulin described above.

### [Example 2]

### (Histone-containing agent for inhibiting bone resorption)

One kilogram of envelope-removed salmon milt (containing 6% nucleic acid) was washed with water and then dehydrated, followed by addition of 3 liters of water and homogenization with a homogenizer. The resulting homogenate was added with a 0.05 N sodium hydroxdide solution to adjust to pH 6 and 20 g of papain was then added and the whole was stirred at 35°C for 2 hours to cleave proteins, followed by retaining at 80°C for 30 minutes to inactivate the enzyme to terminate the cleavage. After the termination of the cleavage, precipitate was removed by centrifugation and the resulting supernatant was then added with 2.5-fold volume of 95% ethanol to precipitate DNA. Subsequently, the resultant was freeze-dried after removal of hydrated ethanol through filtration. Consequently, 65 g of an agent for inhibiting bone resorption of the present invention consisting of DNA powder was obtained. Furthermore, 1 g of the agent for inhibiting bone resorption contained 425 mg of histone.

### [Example 3]

### (Prothrombin-containing agent for promoting osteogenesis and inhibiting bone resorption)

According to a known method (Hashimoto, N., T. Morita, and S. Iwanaga, J. Biochem. (Tokyo), vol. 97, p. 1347-1355, 1985), bovine plasma prothrombin was prepared. In other words, sodium citrate was added to a concentration of 0.01 M and bovine plasma was then obtained by centrifugation. Barium citrate was gradually added to a concentration of 0.1 M in 10 liters of the bovine plasma and then the whole was gently stirred for 1 hour, followed by centrifugation at 4,000 rpm for 10 minutes. The resulting precipitate was washed with a 0.1 M sodium chloride solution containing 0.01 M barium chloride, 1 mM benzamidine hydrochloric acid, and 0.02% sodium azide and then centrifuged. The washing process was repeated twice. The barium precipitate was suspended with 1 litter of 40% saturated ammonium sulfate. Then, the suspension was added with diisoprolyl phosphorofruoridate (DFP) and the whole was stirred overnight. It was centrifuged at 5,000 rpm for 30 minutes to obtain supernatant. The supernant was added with saturated ammonium sulfate to a saturation degree of 67%, followed by centrifugation at 5,000 rpm for 30 minutes to collect the precipitate. The precipitate was dissolved in 100-150 ml of a 0.05 M sodium phosphate buffer (pH 6.0) containing 0.2 M sodium chloride, 1 mM DFP, and 1mM benzamidine hydrochloric acid and then dialyzed overnight with 20 1 of the same buffer. After removal of insoluble components by centrifugation, the remainder was subjected to DEAE-Sephadex A50 chromatography. The resultant was subjected to a DEAE-Sephadex A50 column (200 x 100 mm) equilibrated with a 0.05-M sodium biphosphate buffer (pH 6.0) containing 0.2 M sodium chloride and 1 mM benzamidine hydrochloric acid to gradually increase the concentration of sodium chloride up to 600 mM, thereby eluting an adsorbed protein. Prothrombin was eluted when the sodium chloride concentration reached about 300 mM. The eluent was desalted by dialysis and then subjected to a Blue Sepharose CL-6B column (80 x 200 mm) equilibrated with a 50 mM tris-hydrochloric acid buffer (pH 6.3) containing 0.1 M sodium chloride and 1 mM benzamidine hydrochloric acid. After washing with an equilibrium buffer, the adsorbed protein was eluted with a buffer having a sodium chloride concentration of 4 M. The eluent was desalted by dialysis and then freeze-dried, thereby obtaining 1. 0 g of prothrombin as the active ingredient of the present invention. The 1. 0-gram prothrombine was mixed with 9.0 g of lactose and then formulated into granules, thereby obtaining the agent for promoting osteogenesis and inhibiting bone resorption of the present invention.

Furthermore, any of β₂-microglobulin, histone, complement component 3, monocyte chemotactic protein 1, lysozyme, ribonuclease, cytochrome P-450, plasminogen, transferrin, thrombin, plasmin, complement component 4, and β defensin can be prepared from bovine plasma by the same way as that of prothrombin described above.

### [Example 4]

### (Prothrombin-containing agent for promoting osteogenesis and inhibiting bone resorption)

A prothrombin-containing fraction was prepared from cow milk. A column (5 cm in diameter x 30 cm in height) filled with 400 g of SP Sepharose HP (manufactured by Amersham Biosciences, Co., Ltd.), cation-exchange resin, was washed with a sufficient amount of deionized water, followed by running 40 1 of unsterilized skimmilk (pH 6.7) as a liquid through the column at a flow rate of 25 ml/min. After the liquid had been run, the column was washed sufficiently with deionized water and the protein fraction adsorbed on the resin was then eluted with a 0.02 M carbonic acid buffer (pH 7.0) containing 1.5 M sodium chloride. Subsequently, the eluent was desalted and concentrated by means of a reverse osmosis (RO) membrane and then freeze-dried, thereby obtaining 21 g of protein fraction powder as the agent for promoting osteogenesis and inhibiting bone resorption of the present invention. Furthermore, the protein fraction powder contained 0.01% by weight of prothrombin.

### [Example 5]

### (Ribonuclease-containing agent for inhibiting bone resorption)

Fifty milligrams of ribonuclease (manufactured by Sigma, Co., Ltd.) was added with 93.5 g of hydrated crystalline glucose, 5 g of calcium carbonate, 1 g of sugar ester, and 0.5 g of flavor and mixed together, and the whole was subsequently compressed into tablet, thereby producing the ribonuclease-containing agent for inhibiting bone resorption of the present invention.

### [Example 6]

### (Cytochrome P-450-containing agent for promoting osteogenesis and inhibiting bone resorption)

Ten milligrams of cytochrome P-450 (manufactured by Sigma, Co., Ltd.) was added with 93.5 g of hydrated crystalline glucose, 5 g of calcium carbonate, 1 g of sugar ester, and 0.5 g of flavor and mixed together, and the whole was subsequently compressed into tablet, thereby producing the cytochrome P-450-containing agent for promoting osteogenesis and inhibiting bone resorption of the present invention.

### [Example 7]

### (Agent for accelerating osteogenesis, containing complement component 4 and β defensin)

Ten liters of skimmed cow milk was run through an S-Sepharose column (manufactured by Pharmacia, Co., Ltd.), followed by washing sufficiently with 20 mM phosphate buffer (pH 7.0). While a ratio of a phosphate buffer (pH 8.5) containing 1.5 M salt was raised to 100% within 1 hour, the adsorbed protein was subjected to gradient elution and 0.5 mg of complement component 4 was then collected at a position corresponding to an elution time of 15 minutes and 0.3 mg of β defensin at a position corresponding to an elution time of 20 minutes.

Complement component 4 and β defensin thus obtained can be directly used as the agents for promoting osteogenesis of the present invention.

### [Example 8]

### (Complement component 4-containing agent for promoting osteogenesis)

One milligram of complement component 4 (Complement component C4, C8195 manufactured by Sigma, Co., Ltd.) was added with 93.4 g of hydrated crystalline glucose, 5 g of calcium carbonate, 1 g of sugar ester, and 0.5 g of flavor and mixed together, and the whole was subsequently compressed into tablet, thereby producing the complement component 4-containing agent for promoting osteogenesis of the present invention.

### [Example 9]

### (β defensin-containing agent for promoting osteogenesis)

One milligram of β defensin (β-Defensin-1, 4337-s, manufactured by Peptide Institute, Inc.) was added with 93.4 g of hydrated crystalline glucose, 5 g of calcium carbonate, 1 g of sugar ester, and 0.5 g of flavor and mixed together, and the whole was subsequently compressed into tablet, thereby producing the β defensin-containing agent for promoting osteogenesis of the present invention.

### [Example 10]

### (Agent for promoting osteogenesis, containing complement component 4 and β defensin)

One milligram of complement component 4 (Complement component C4, C8195, manufactured by Sigma, Co., Ltd.) and one milligram of β defensin (β-Defensin-2, 4338-s, manufactured by Peptide Institute, Inc.) were added with 93.4 g of hydrated crystalline glucose, 5 g of calcium carbonate, 1 g of sugar ester, and 0.5 g of flavor and mixed together, and the whole was subsequently compressed into tablet, thereby producing the agent for promoting osteogenesis containing complement component 4 and β defensin of the present invention.

### [Example 11]

### (β₂-microglobulin-containing drink for inhibiting bone resorption)

After raw materials had been mixed with the formulation shown in Table 4, the mixture was loaded in a container and then heat-sterilized, thereby producing the β₂-microglobulin-containing drink for inhibiting bone resorption of the present invention.

**Table 4**

| | | |
|---|---|---|
| Saccharide isomerate | 15.0 | (wt%) |
| Fruit juice | 10.0 | |
| Citric acid β₂microglobulin | 0.5 | |
| (Example 1) | 0.05 | |
| Flavor | 0.1 | |
| Calcium | 0.5 | |
| Water | 73.85 | |

### [Example 12]

### (Lysozyme-containing biscuit for inhibiting bone resorption)

Raw materials were mixed with the formulation shown in Table 5 and dough was then prepared and formed into a shape, followed by roasting to produce the lysozyme-containing biscuit for inhibiting bone resorption of the present invention.

**Table 5**

| | | |
|---|---|---|
| Flour | 50.35 | (wt%) |
| Sugar | 20.0 | |
| Salt | 0.5 | |
| Margarine | 12.5 | |
| Egg | 12.1 | |
| Water | 3.7 | |
| Sodium bicarbonate | 0.1 | |
| Ammonium bicarbonate | 0.2 | |
| Calcium carbonate | 0.5 | |
| Lysozyme (manufactured by Sigma Corp.) | 0.05 | |

### [Example 13]

### (Complement component 3-containing jelly for inhibiting bone resorption)

After raw materials had been mixed with the formulation shown in Table 6, the mixture was loaded in a container and heat-sterilized, thereby producing the complement component 3-containing jelly for inhibiting bone resorption of the present invention.

**Table 6**

| | | |
|---|---|---|
| Fructose | 20.0 | (wt%) |
| Granulated sugar | 15.0 | |
| Starch syrup | 5.0 | |
| Agar | 1.0 | |
| Complement component 3 (manufactured by Sigma Corp.) | 0.05 | |
| Flavor | 0.11 | |
| Calcium | 0.1 | |
| Water | 58.74 | |

### [Example 14]

### (Histone-containing processed cheese for inhibiting bone resorption)

After raw materials had been mixed with the formulation shown in Table 7, the mixture was emulsified at 85 °C, thereby producing the histone-containing processed cheese for inhibiting bone resorption of the present invention.

**Table 7**

| | | |
|---|---|---|
| Gouda cheese | 43.0 | (wt%) |
| Cheddar cheese | 43.5 | |
| Sodium citrate | 2.0 | |
| Histone H1/2A (manufactured by Sigma Corp.) | 0.05 | |
| Milk derived calcium | 1.0 | |
| Water | 10.45 | |

### [Example 15]

### (Yogurt for inhibiting bone resorption, which contains monocyte chemotactic protein 1)

After heat-sterilization of 12-weight% reduced skimmilk at 90°C for 20 minutes, *Lactobacillus acidophilus* and *Streptococcus thermophilus* were independently inoculated to obtain two different starter cultures, and both of them were mixed in equal proportions. Subsequently, after rawmaterials had been mixed with the formulation shown in Table 8, the mixture was fermented, thereby producing the yogurt for inhibiting bone resorption of the present invention, which contained monocyte chemotactic protein 1.

**Table 8**

| | | |
|---|---|---|
| Yogurt mixture | 96.95 | (wt%) |
| Starter culture | 3.0 | |
| Monocyte chemotactic protein 1 (manufactured by Biotech Corp.) | 0.05 | |

### [Example 16]

### (Infant formula for inhibiting bone resorption, which contains β₂-microglobulin and complement component 3)

Raw materials were mixed with the formulation shown in Table 9, thereby producing the infant formula milk for inhibiting bone resorption of the present invention, which contained β₂-microglobulin and complement component 3.

**Table 9**

| | | |
|---|---|---|
| Skimmilk powder | 75.85 | (wt%) |
| Whey protein concentrate | 2.36 | |
| Lactose | 13.86 | |
| Mineral mixture | 0.32 | |
| Fat including fat-soluble vitamins | 7.53 | |
| β₂-microglobulin (manufactured by Sigma Corp.) | 0.03 | |
| Complement component 3 (manufactured by Sigma Corp.) | 0.05 | |

### [Example 17]

### (Histone-containing feed for dog-breeding for inhibiting bone resorption)

Raw materials were mixed with the formulation shown in Table 10, thereby producing the histone-containing feed for dog-breeding (dog food) for inhibiting bone resorption of the present invention.

**Table 10**

| | | |
|---|---|---|
| Soybean cake | 12.0 | (wt%) |
| Skimmilk powder | 13.95 | |
| Soybean oil | 4.0 | |
| Corn oil | 2.0 | |
| Palm oil | 28.0 | |
| Corn starch | 15.0 | |
| Flour | 9.0 | |
| Wheat bran | 2.0 | |
| Vitamin mixture | 9.0 | |
| Mineral mixture | 2.0 | |
| Cellulose | 3.0 | |
| Histone H1/2A (manufactured by Sigma Corp.) | 0.05 | |

### [Example 18]

### (Juice for promoting osteogenesis and inhibiting bone resorption, which contains transferrin and cytochrome P-450)

Raw materials were mixed with the formulation shown in Table 11, and then the mixture was loaded in a container and heat-sterilized, thereby producing the juice for promoting osteogenesis and inhibiting bone resorption of the present invention, which contained transferrin and cytochrome P-450.

**Table 11**

| | | |
|---|---|---|
| Saccharide isomerate | 15.0 | (wt%) |
| Fruit juice | 10.0 | |
| Citric acid | 0.5 | |
| Transferrin(manufactured by Funakoshi Co., Ltd) | 0.02 | |
| Cytochrome P-450 (manufactured by Sigma Corp.) | 0.03 | |
| Flavor | 0.1 | |
| Calcium | 0.5 | |
| Water | 73.85 | |

### [Example 19]

### (Plasminogen-containing biscuit for promoting osteogenesis and inhibiting bone resorption)

Raw materials were mixed with the formulation shown in Table 12 using commercially-available plasminogen and dough was then prepared and formed into a shape, followed by roasting to produce the biscuit for promoting osteogenesis and inhibiting bone resorption of the present invention.

**Table 12**

| | | |
|---|---|---|
| Flour | 50.0 | (wt%) |
| Sugar | 20.0 | |
| Salt | 0.5 | |
| Margarine | 12.5 | |
| Egg | 12.1 | |
| Water | 4.05 | |
| Sodium hydrocarbonate | 0.1 | |
| Ammonium bicarbonate | 0.2 | |
| Calcium carbonate | 0.5 | |
| Plasminogen (manufactured by Sigma Corp.) | 0.05 | |

### [Example 20]

### (Plasmin-containing biscuit for promoting osteogenesis and inhibiting bone resorption)

Raw materials were mixed with the formulation shown in Table 13 using commercially-available plasmin, thereby producing the biscuit for promoting osteogenesis and inhibiting bone resorption of the present invention.

**Table 13**

| | | |
|---|---|---|
| Flour | 50.0 | (wt%) |
| Sugar | 20.0 | |
| Salt | 0.5 | |
| Margarine | 12.5 | |
| Egg | 12.1 | |
| Water | 4.05 | |
| Sodium hydrocarbonate | 0.1 | |
| Ammonium bicarbonate | 0.2 | |
| Calcium carbonate | 0.5 | |
| Plasmin (manufactured by Sigma Corp.) | 0.05 | |

### [Example 21]

### (Transferrin-containing jelly for promoting osteogenesis and inhibiting bone resorption)

Raw materials were mixed with the formulation shown in Table 14 and then filled in a container, followed by heat sterilization. Consequently, the transferrin-containing jelly for promoting ostogenesis and inhibiting bone resorption of the present invention was produced.

**Table 14**

| | | |
|---|---|---|
| Fructose | 20.0 | (wt%) |
| Granulated sugar | 15.0 | |
| Starch syrup | 5.0 | |
| Agar | 1.0 | |
| Transferrin(manufactured by Funakoshi Co., Ltd) | 0.05 | |
| Flavor | 0.11 | |
| Calcium carbonate | 0.45 | |
| Water | 58.39 | |

### [Example 22]

### (Prothrombin-containing processed cheese for promoting osteogenesis and inhibiting bone resorption)

Raw materials were mixed with the formulation shown in Table 15 using commercially-available prothrombin and then emulsified at 85°C, thereby producing the prothrombin-containing processed cheese for promoting osteogenesis and inhibiting bone resorption of the present invention.

**Table 15**

| | | |
|---|---|---|
| Gouda cheese | 43.0 | (wt%) |
| Cheddar cheese | 43.5 | |
| Sodium citrate | 2.0 | |
| Prothrombin (manufactured by Sigma Corp.) | 0.05 | |
| Milk derived calcium | 1.0 | |
| Water | 10.45 | |

### [Example 23]

### (Thrombin-containing processed cheese for promoting osteogenesis and inhibiting bone resorption)

Raw materials were mixed with the formulation shown in Table 16 using commercially-available thrombin, thereby producing the thrombin-containing processed cheese for promoting osteogenesis and inhibiting bone resorption of the present invention.

**Table 16**

| | | |
|---|---|---|
| Gouda cheese | 43.0 | (wt%) |
| Chedda cheese | 43.5 | |
| Sodium citrate | 2.0 | |
| Thrombin (manufactured by Sigma Corp.) | 0.05 | |
| Milk derived calcium | 1.0 | |
| Water | 10.45 | |

### [Example 24]

### (Transferrin-containing yogurt for promoting osteogenesis and inhibiting bone resorption)

After heat-sterilization of 12-weight% reconstituted skimmilk at 90°C for 20 minutes, *Lactobacillus acidophilus* and *Streptococcus thermophilus* were independently inoculated to obtain two different starter cultures, and both of them were mixed in equal proportions. Subsequently, after raw materials had been mixed with the formulation shown in Table 17, the mixture was fermented, thereby producing the transferrin-containing yogurt for promoting osteogenesis and inhibiting bone resorption of the present invention.

**Table 17**

| | | |
|---|---|---|
| Yoghurt mixture | 97.0 | (wt%) |
| Starter culture | 2.95 | |
| Transferrin (manufactured by Funakoshi Co., Ltd | 0.05 | |

### [Example 25]

### (Prothrombin-containing infant formula milk for promoting osteogenesis and inhibiting bone resorption)

Raw materials were mixed with the formulation shown in Table 18 using prothrombin obtained in Example 3, thereby producing the infant formula for promoting osteogenesis and inhibiting bone resorption of the present invention.

**Table 18**

| | | |
|---|---|---|
| Skimmilk powder | 75.85 | (wt%) |
| Whey protein concentrate | 2.36 | |
| Lactose | 13.86 | |
| Mineral mixture | 0.32 | |
| Fat including fat-soluble vitamins | 7.53 | |
| Prothrombin (Example 3) | 0.08 | |

### [Example 26]

### (Thrombin-containing infant formula for promoting osteogenesis and inhibiting bone resorption)

Raw materials were mixed with the formulation shown in Table 19 using commercially-available thrombin, thereby producing the infant formula for promoting osteogenesis and inhibiting bone resorption of the present invention.

**Table 19**

| | | |
|---|---|---|
| Skimmilk powder | 75.85 | (wt%) |
| Whey protein concentrate | 2.36 | |
| Lactose | 13.86 | |
| Mineral mixture | 0.32 | |
| Fat including fat-soluble vitamins | 7.53 | |
| Thrombin (manufactured by Sigma Corp.) | 0.08 | |

### [Example 27]

### (Plasminogen-containing feed for dog-breeding for promoting osteogenesis and inhibiting bone resorption)

Raw materials were mixed with the formulation shown in Table 20 using commercially-available plasminogen, thereby producing the feed for dog-breeding (dog food) for promoting osteogenesis and inhibiting bone resorption of the present invention.

**Table 20**

| | | |
|---|---|---|
| Soybean cake | 12.0 | (wt%) |
| Skimmilk powder | 14.5 | |
| Soybean oil | 4.0 | |
| Corn oil | 2.0 | |
| Palm oil | 28.0 | |
| Corn starch | 14.45 | |
| Flour | 9.0 | |
| Wheat bran | 2.0 | |
| Vitamin mixture | 9.0 | |
| Mineral mixture | 2.0 | |
| Cellulose | 3.0 | |
| Plasminogen (manufactured by Sigma Corp.) | 0.05 | |

### [Example 28]

### (Plasmin-containing feed for dog-breeding for promoting osteogenesis and inhibiting bone resorption)

Raw materials were mixed with the formulation shown in Table 21 using commercially-available plasmin, thereby producing the feed for dog-breeding (dog food) for promoting osteogenesis and inhibiting bone resorption of the present invention.

**Table 21**

| | | |
|---|---|---|
| Soybean cake | 12.0 | (wt%) |
| Skimmilk powder | 14.5 | |
| Soybean oil | 4.0 | |
| Corn oil | 2.0 | |
| Palm oil | 28.0 | |
| Corn starch | 14.45 | |
| Flour | 9.0 | |
| Wheat bran | 2.0 | |
| Vitamin mixture | 9.0 | |
| Mineral mixture | 2.0 | |
| Cellulose | 3.0 | |
| Plasmin manufactured by Sigma Corp.) | 0.05 | |

### [Example 29]

### (Milk beverage for promoting osteogenesis, which contains complement component 4 and β defensin)

Rawmilk was homogenized at a homogeneous pressure of 120 kg/cm². Subsequently, the raw milk heat-sterilized at 75°C for 15 seconds was added with complement component 4 (Complement component C4, C8195, manufactured by Sigma, Co., Ltd.) under sterile conditions to a concentration of 10 mg/l, and then loaded in a glass bottle of 100 ml in volume, thereby producing the milk beverage for promoting osteogenesis of the present invention, which contained complement component 4 and β defensin.

### [Example 30]

### (β defensin-containing milk beverage for promoting osteogenesis)

Raw milk was homogenized at a homogeneous pressure of 120 kg/cm². Subsequently, the raw milk heat-sterilized at 75°C for 15 seconds was added with β defensin (β-Defensin-1, 4337-s, manufactured by Peptide Institute, Inc.) under sterile conditions to a concentration of 10 mg/l, and then loaded in a glass bottle of 100 ml in volume, thereby producing the β defensin-containing milk beverage for promoting osteogenesis of the present invention.

### [Example 31]

### (Complement component 4-containing jelly for promoting osteogenesis)

Raw materials were mixed at a ratio of fructose 20.0 (% by weight), granulated sugar 15.0 (% by weight), starch syrup 5.0 (% by weight), agar 1.0 (% by weight), flavor 0.11 (% by weight), calcium 0.1 (% by weight), and water 58.39 (% by weight), and then heat-sterilized. Subsequently, the resultant was added with complementcomponent 4 (Complement component C4, C8195, manufactured by Sigma, Co., Ltd.) under sterile conditions to a concentration of 10 mg/l, and then loaded in a container, thereby producing the complement component 4-containing jelly for promoting osteogenesis of the present invention.

### [Example 32]

### (β defensin-containing infant formula for promoting osteogenesis)

Raw materials were mixed at a ratio of skimmilk 75.61 (% by weight), whey protein concentrate 2. 36 (% by weight), lactose 13. 86 (% by weight), mineral mixture 0.32 (% by weight), water-soluble vitamin mixture 0.32 (% by weight), and fat 7.53 (% by weight) containing fat-soluble vitamins, and then sterilized, followed by concentration and spray drying. Consequently, raw powder of infant formula was produced. Subsequently, the powder was added and mixed with β defensin (β-Defensin-2, 4338-s, manufactured by Peptide Institute, Inc.) under sterile conditions to a concentration of 10 mg/kg, thereby producing the β defensin-containing infant formula for promoting osteogenesis of the present invention.

### [Example 33]

### (Complement component 4-containing dog food for promoting osteogenesis)

Raw materials were mixed at a ratio of soybean cake 12.0 (% by weight), skimmilk powder 14.0 (% by weight), soybean oil 4.0 (% by weight), corn oil 2.0 (% by weight), palm oil 28.0 (% by weight), corn starch 15.0 (% by weight), wheat flour 9.0 (% by weight), bran 2. 0 (% by weight), vitamin mixture 9.0 (% by weight), mineral mixture 2. 0 (% by weight), and cellulose 3. 0 (% by weight), and then sterilized and cooled. Subsequently, the resultant was added with complement component 4 (Complement component C4, C8195, manufactured by Sigma, Co., Ltd.) under sterile conditions to a concentration of 10 mg/l and then formed into a shape, thereby producing the complement component 4-containing feed for dog-breeding (dog food) for promoting osteogenesis of the present invention.

## Claims

1. An agent for promoting osteogenesis and/or inhibiting bone resorption, comprising any one or more of β₂-microglobulin, histone, complement component 3, monocyte chemotactic protein 1, lysozyme, ribonuclease, prothrombin, cytochrome P-450, plasminogen, transferrin, thrombin, plasmin, complement component 4, and β defensin, and enzymatic digestion products thereof as an active ingredient.

2. An agent for inhibiting bone resorption, comprising any one or more of β₂-microglobulin, histone, complement component 3, monocyte chemotactic protein 1, lysozyme, ribonuclease, prothrombin, cytochrome P-450, plasminogen, transferrin, thrombin, and plasmin, and enzymatic digestion products thereof.

3. An agent for promoting osteogenesis and inhibiting bone resorption, comprising any one or more of prothrombin, cytochrome P-450, plasminogen, transferrin, thrombin, plasmin, and enzymatic digestion products thereof.

4. An agent for promoting osteogenesis, comprising any one or more of complement component 4, β defensin, and enzymatic digestion products thereof.

5. A food, drink, drug, or feed for promoting osteogenesis, comprising any one or more of β₂-microglobulin, histone, complement component 3, monocyte chemotactic protein 1, lysozyme, ribonuclease, prothrombin, cytochrome P-450, plasminogen, transferrin, thrombin, and plasmin, and enzymatic digestion products thereof.

6. A food, drink, drug, or feed for promoting osteogenesis and/or inhibiting bone resorption, comprising any one or more of prothrombin, cytochrome P-450, plasminogen, transferrin, thrombin, plasmin, and enzymatic digestion products thereof.

7. A food, drink, drug, or feed for promoting osteogenesis, comprising any one or more of complement component 4, β defensin, and enzymatic digestion products thereof.
